# EUROPEAN PATENT SPECIFICATION

(11) **EP 0 367 862 B1**
(45) Date of publication and mention of the grant of the patent: **21.04.1993**
(21) Application number: 88118857.7
(22) Date of filing: 11.11.1988
(51) Int. Cl.: C07D 273/00

(54) **Perfluoro-amino-oxaziridines and process for their preparation**
Perfluoramino-oxaziridine und Verfahren zu ihrer Herstellung
Perfluoroamino-oxaziridines et procédé pour leur préparation

(43) Date of publication of application: 16.05.1990
(73) Proprietor: AUSIMONT S.r.l., I-20100 Milano (IT)
(72) Inventor: Navarrini, Walter, 1-20010 Boffalora Ticino Milan (IT); Desmarteau, Darryl D., Clemson University South Carolina 29631 (US)
(74) Representative: Barz, Peter, Dr.

(56) References cited:
- FR-A- 2 216 285
- GB-A- 743 940
- US-A- 4 287 128
- TETRAHEDRON LETTERS, no. 7, February 1972, pages 633,634, Pergamon Press, Oxford, GB; J.-P. SCHIRMANN et al.: "Chimie des peroxydes en milieu basique. I - Oxydation des bases de schiff en oxaziridines avec le peroxyde d'hydrogène et un nitrile"

## Description

The present invention concerns a new type of perfluoro-oxaziridines and a process for their preparation.

The perfluoro-oxaziridines of the present invention are characterized by the presence of an amine group in position 3 on the oxaziridine ring according to the following formula: 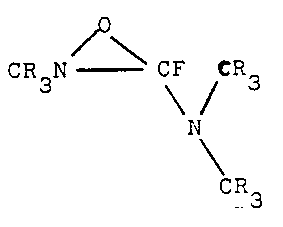
wherein each R is independently either F or a perfluoroalkyl radical having from 1 to 10 carbon atoms.

The perfluoro-oxaziridines are a little-known class of organic compounds.

The simplest member: 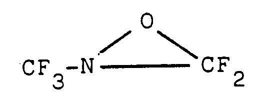
has been described by Falardeau and Des Marteau in the "Journal of American Chemical Society", 98, page 3529 (1976).

The process for the preparation of said compound comprises the synthesis of a potentially explosive hydroperoxide which is difficult to prepare. This process is, however, not effective for the preparation of oxaziridines substituted with perfluoroalkyl groups.

US-A-4 287 128 describes oxaziridines of the general formula: 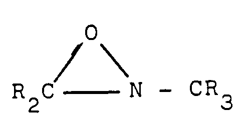
wherein R may be independently either fluorine or a perhalogenated alkyl radical having up to 10 carbon atoms with the exclusion of

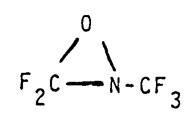

The process for the preparation of the perfluoroalkyl oxaziridines comprises reacting a perfluoroalkylimine, as a starting compound, with gaseous chlorine in the presence of a carbonate or bicarbonate of an alkali or alkaline earth metal according to the reaction:

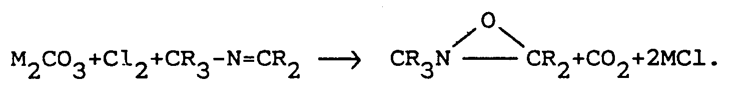

The reaction temperature of such a process is comprised between -20°C and +100°C.

According to this process it is indispensable that, in the reaction mixture, traces of water be present so as to catalyze the described reaction.

It is important to note that there has to be sufficient water to form the hypochlorite with the reacting chlorine, although it is not specified how much water can possibly be added. It should, in fact, be noted that the oxaziridines described in US-A-4 287 128 may easily be hydrolyzed by H₂O.

These compounds are particularly reactive and may easily be hydrolyzed to aldehydes and β-alkylhydroxylamines, as is suggested by the cited US patent.

It is, for instance, not possible to prepare the first member of the series, starting from CF₃-N=CF₂, by employing the method described above. For the preparation of the first member, the complex Des Marteau method described above is quite effective but shows the previously mentioned drawbacks.

The process described in US-A-4 287 128, moreover, has a number of disadvantages such as, for instance, the regulation (adjustment) of the percentage of water that acts as a catalyst and the proper quantity of gaseous chlorine to be introduced into the reaction mixture, depending on the active surface of the catalyst. In fact, in the presence of gaseous chlorine, the catalyst tends to easily deactivate itself during the reaction.

US-A-3 358 003 describes a similar process for the preparation of epoxides from the corresponding perfluorinated olefins in the presence of a base and hydrogen peroxide.

It has, surprisingly, been found that, notwithstanding the easy hydrolysis of the oxaziridines, it is possible to prepare said compounds by reacting perfluoroalkylimines with a base, i.e., carbonates or bicarbonates of alkali or alkaline earth metals, in the presence of H₂O₂ and a solvent such as acetonitrile or diglyme, thus overcoming all the drawbacks of US-A-4 287 128.

Moreover, as far as US-A-3 358 003 is concerned, it is important to note the diversity of the epoxy group in respect of oxaziridines, as well as the fact that solvents especially recommended for the preparation of the epoxides are aliphatic alcohols, acetone or acetaldehyde which, on the contrary, do not favour the conversion of oxaziridines, for the preparation of which the use of solvents such as acetonitrile or diglyme is particularly recommended.

Thus, a further object of the present invention is to provide a process for the preparation of perfluoro-oxaziridines of general formula: 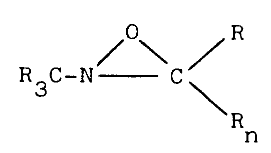
wherein each R is independently either F or a perfluoroalkyl group having from 1 to 10 carbon atoms, while Rₙ may be either a perfluoroalkyl group having from 1 to 10 carbon atoms or an amino group of the type: 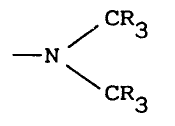
wherein R has the meaning indicated above, which process comprises reacting a perfluoroimine of the type 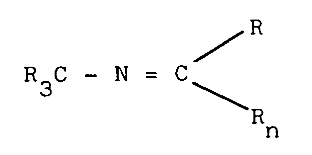
wherein R and Rₙ have the meaning indicated above, with H₂O₂ in the presence of carbonates or bicarbonates of alkali metals or alkaline earth metals in a dipolar aprotic solvent, at a temperature of between -50°C and +50°C.

Preferably, use is made of diglyme or acetonitrile as dipolar aprotic solvents. The contact times are not limited in the present invention although it is preferable to conduct the reaction at a reaction time of between 1 and 60 minutes, depending on the reaction temperature chosen.

With the process of the present invention it is possible to prepare any perfluoro-oxaziridine having F and perfluoroalkyl terminal groups, with the exception of:

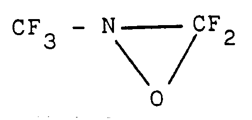

Among the oxaziridines prepared by means of the process of the present invention, the perfluoroamino-oxaziridines of general formula 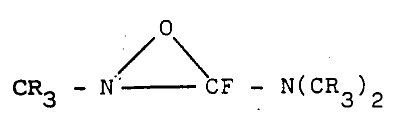
are preferred, wherein each R may be either fluorine or a perfluoroalkyl group having up to 10 carbon atoms.

Particularly preferred is 2-(trifluoromethyl)-3-fluoro-3-bis(trifluoromethyl)amino-oxaziridine of the formula:

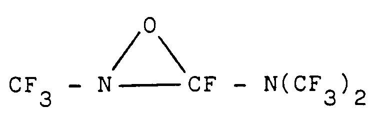

The oxaziridines of the present invention are useful as intermediates in the prepartion of nitrons. Moreover, they may form complexes with ions of transition metals and be used as catalysts in the photochemical polymerization of ethylenic monomers.

Furthermore, they may polymerize and copolymerize, forming liquids with a high thermal stability.

The following examples merely serve to illustrate the invention and do not limit it in any way.

### Example 1

9 g of sodium bicarbonate, 100 ml of acetonitrile, 6.3 ml of hydrogen peroxide at 50% concentration and, by means of standard distillation techniques, 7.9 g of 1-bis(trifluoromethyl)aminotetrafluoro-2-aza-1-propylene, were charged into an 0.5-litre glass flask fitted with a magnetic stirrer.

The reactor thus charged was maintained a a temperature of 0°C for 1 hour under stirring. The crude reaction product was then distilled at a pressure of 1.3·10⁻⁴ kPa (10⁻³ Torr). The vapour coming from the distillation boiler was made to pass through cooled traps kept at a temperature of -75°C and -120°C, respectively.

The trap kept at -75°C contained condensed acetonitrile and the water, while the trap kept at -120°C contained 4.2 g of the desired product, 2-(trifluoromethyl)-3-fluoro-3-bis(trifluoromethyl)amino-oxaziridine, substantially in pure form. The CO₂, on the contrary, was removed by dynamical vacuum during the distillation itself.

The conversion of the starting product was complete. The yield of the reaction, defined as the ratio between moles of desired product and moles of reacted starting product, amounted to 50%.

### Physical properties and characteristics of the product:

| | |
|---|---|
| Boiling point | 41°C |
| Melting point | -130°C |

Infrared spectrum main absorption bands:
cm⁻¹ (intensity), 1422 (s), 1358 (vs), 1307 (vs), 1224 (vs), 1198 (vs), 1052 (w).
vs = very strong; s = strong; m = medium; w = weak.
NMR spectrum ¹⁹F: (internal reference CFCl₃, solv. CDCl₃).

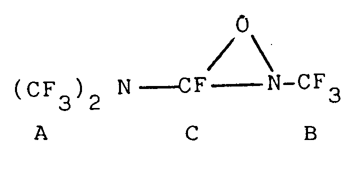

| | |
|---|---|
| δFA = (d) - 56.6 ppm | ^{J}AC = 6.1 Hz |
| δFB = (d) - 65.7 ppm | ^{J}BC = 18.3 Hz |
| δFC = (m) - 100 ppm | |

### Example 2

The procedure of example 1 was followed but sodium carbonate was used instead of sodium bicarbonate.

9 g of sodium carbonate, 100 ml acetonitrile, 6.3 ml of hydrogen and, through standard distillation techniques, 7.9 g of 1-bis(trifluoromethyl)aminotetrafluoro-2-aza-1-propylene were charged into an 0.5-litre glass flask fitted with a magnetic stirrer.

The reactor thus charged was maintained at 0°C for 1 hour under stirring. The reaction product was then distilled at a pressure of 1.3·10⁻⁴ kPa (10⁻³ Torr) in the same way as in example 1. The -120°C trap contained 4.6 g of substantially pure 2-(trifluoromethyl)-3-fluoro-3-bis(trifluoromethyl)amino-oxaziridine .

The conversion of the starting product was complete and the yield, defined as in example 1, amounted to 55%.

### Example 3

4.5 g of sodium bicarbonate, 50 ml diglyme, 3 ml of 50% hydrogen peroxide and, through standard distillation techniques, 3.8 g of 1-bis(trifluoromethyl)aminotetrafluoro-2-aza-1-propylene were charged into an 0.25-litre glass flask fitted with a magnetic stirrer.

The reactor thus charged was maintained at 0°C for 15 minutes under constant stirring. The crude reaction product was then distilled at a pressure of 1.3·10⁻⁴ kPa (10⁻³ Torr) in the same way as in example 1. The trap at -75°C contained diglyme and water, while the trap at -120°C contained 0.65 g of 2-(trifluoromethyl)-3-fluoro-3-bis(trifluoromethyl)amino-oxaziridine, substantially in pure form.

The conversion of the starting product was complete and the yield, defined as in example 1, amounted to 16%.

### Example 4

The procedure of example 3 was repeated but the operation was carried out in the absence of a basic catalyst.

50 ml of diglyme, 3 ml of 50% hydrogen peroxide and, through standard distillation techniques, 3.8 g of 1-bis(trifluoromethyl)amino-tetrafluoro-2-aza-1-propylene were charged into a 250 ml glass flask fitted with a magnetic stirrer.

The reactor thus charged was kept at 0°C for 15 minutes under constant stirring. The crude product was distilled at a pressure of 1.3·10⁻⁴ kPa (10⁻³ Torr) as in example 3. The trap at -120°C contained 0.22 g of substantially pure 2-(trifluoromethyl)-3-fluoro-3-bis(trifluoromethyl)amino-oxaziridine.

The conversion of the starting reaction product amounted to 60% while the yield was 5%.

### Example 5

2 g of sodium bicarbonate, 20 ml of acetonitrile, 1 ml of H₂O₂ (50% by weight) and 5 g of perfluoro(6-aza-5-undecene):

CF₃-(CF₂)₄-N=CF-(CF₂)₃-CF₃

were charged into a 100 ml glass flask fitted with a magnetic stirrer.

The reactor thus charged was maintained at a temperature of 0°C for 1 hour under constant stirring. The crude reaction product was distilled at a pressure of 1.3·10⁻⁴ kPa (10⁻³ Torr). The vapours coming from the distillation boiler were then passed through cooled traps kept at a temperature of -40°C and -120°C, respectively.

The -120°C trap contained condensed acetonitrile and some water while the trap kept at -40°C contained 2 g of 2-(perfluoropentyl)-3-(perfluorobutyloxaziridine):

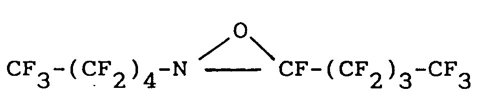

The oxaziridine contained a few ppm of water. It was easily dried over Na₂SO₄. The CO₂ was removed during distillation. The conversion of the starting product was complete.

The yield, defined as in example 1, was 38%.

## Claims

1. Perfluoro-oxaziridines, characterized by the presence of an amine group in position 3 of the oxaziridine ring of the general formula: 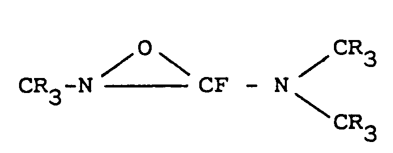 wherein each R independently is either F or a perfluoroalkyl group having from 1 to 10 carbon atoms.

2. A perfluoro-oxaziridine of claim 1, characterized in that R is F.

3. A process for the preparation of perfluoro-oxaziridines of the general formula: 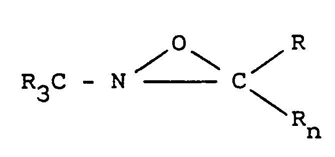 wherein each R independently is either F or a perfluoroalkyl group having from 1 to 10 carbon atoms while Rₙ may be either a perfluoroalkyl group having from 1 to 10 carbon atoms or an amine group of the type: 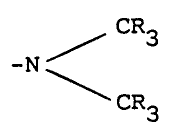 which comprises reacting a perfluoroimine of the general formula: 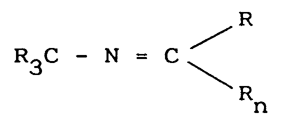 with H₂O₂ in the presence of a carbonate or bicarbonate of an alkali or alkaline earth metal in a dipolar aprotic solvent at a temperature of from -50°C to +50°C.

4. The process of claim 3, wherein the dipolar aprotic solvent is diglyme or acetonitrile.

## Patentansprüche

1. Perfluoroxaziridine, gekennzeichnet durch die Anwesenheit einer Amingruppe in 3-Stellung des Oxaziridinrings der allgemeinen Formel: 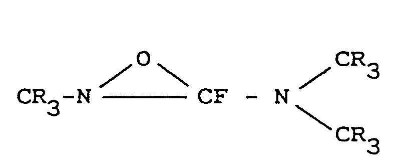 worin jedes R unabhängig entweder F oder eine Perfluoralkylgruppe mit 1 bis 10 Kohlenstoffatomen darstellt.

2. Perfluoroxaziridin nach Anspruch 1, dadurch gekennzeichnet, daß R F ist.

3. Verfahren zur Herstellung von Perfluoroxaziridinen der allgemeinen Formel: 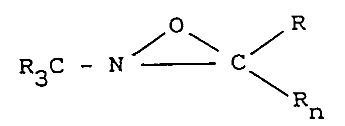 worin jedes R unabhängig entweder F oder eine Perfluoralkylgruppe mit 1 bis 10 Kohlenstoffatomen ist, wohingegen R₂ entweder eine Perfluoralkylgruppe mit 1 bis 10 Kohlenstoffatomen oder eine Amingruppe des Typs: 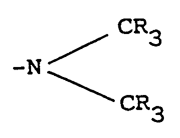 sein kann, welches umfaßt das Umsetzen eines Perfluorimins der allgemeinen Formel: 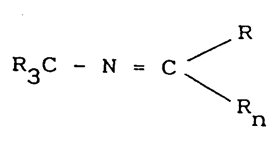 mit H₂O₂ in Anwesenheit eines Carbonats oder Bicarbonats eines Alkali- oder Erdalkalimetalls in einem dipolaren aprotischen Lösungsmittel bei einer Temperatur von -50°C bis + 50°C.

4. Verfahren nach Anspruch 3, worin das dipolare aprotische Lösungsmittel Diglyme oder Acetonitril ist.

## Revendications

1. Perfluorooxaziridines, caractérisées par la présence d'un groupe amine en position 3 du cycle oxaziridine, représenté par la formule générale: 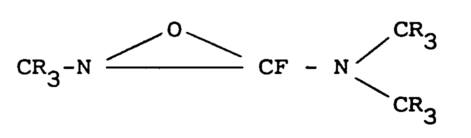 dans laquelle chaque R est indépendamment un atome de fluor ou un groupe perfluoroalkyle ayant 1 à 10 atomes de carbone.

2. Perfluorooxaziridines suivant la revendication 1, caractérisées en ce que R est un atome de fluor.

3. Procédé pour la préparation de perfluorooxaziridines de formule générale: 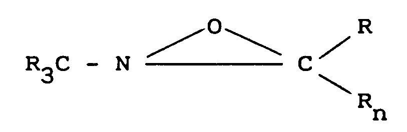 dans laquelle chaque R est indépendamment un atome de fluor ou un groupe perfluoroalkyle ayant de 1 à 10 atomes de carbone, tandis que Rₙ peut être soit un groupe perfluoroalkylé ayant de 1 à 10 atomes de carbone, soit un groupe amine du type: 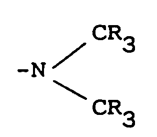 qui comprend la réaction d'une perfluoroimine de formule générale: 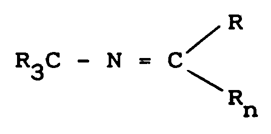 avec du H₂O₂ en présence d'un carbonate ou carbonate acide d'un métal alcalin ou alcalino-terreux dans un solvant aprotique dipolaire à une température de -50°C à °50°C.

4. Procédé suivant la revendication 3, dans lequel le solvant aprotique dipolaire est la diglyme ou l'acétonitrile.
